(19) Europäisches Patentamt / European Patent Office / Office européen des brevets

(11) **EP 2 572 703 A1**

(12) **EUROPEAN PATENT APPLICATION**

(43) Date of publication:
27.03.2013 Bulletin 2013/13

(51) Int Cl.:
*A61K 9/00* (2006.01)  *A61K 9/20* (2006.01)
*A61K 31/407* (2006.01)

(21) Application number: 11182256.5

(22) Date of filing: 21.09.2011

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: Hexal AG
83607 Holzkirchen (DE)

(72) Inventor: **The designation of the inventor has not yet been filed**

(74) Representative: **Englmeier, Ludwig**
**c/o Sandoz International GmbH**
**Global Patents Department**
**Industriestrasse 25**
**83607 Holzkirchen (DE)**

(54) **Compressed oral dosage form for asenapine maleate**

(57)    The present invention relates to a compressed pharmaceutical dosage form intended for sublingual or buccal administration and capable of being rapidly disintegrated, the compressed pharmaceutical dosage form containing asenapine maleate in a microcrystalline monoclinic form. It further relates to a method of preparing the same and to a container comprising the dosage form.

**Description**

**SUBJECT OF THE INVENTION**

The present invention relates to a compressed pharmaceutical dosage form intended for sublingual or buccal administration and capable of being rapidly disintegrated, the compressed pharmaceutical dosage form containing asenapine maleate in a microcrystalline monoclinic form. It further relates to a method of preparing the same and to a container comprising the dosage form.

**BACKGROUND OF THE INVENTION**

Asenapine (INN, Org 5222, trade names: Saphris®, Sycrest®) is an atypical antipsychotic developed for the treatment of schizophrenia and acute mania associated with bipolar disorder. Asenapine (i.e. *trans*-5-Chloro-2,3,3a,12b-tetrahydro-2-methyl-1*H*-dibenz[2,3:6,7]oxepino[4,5-c]pyrrole) is a pyrrolidino tetracyclic compound with potent dopamine, serotonin and adrenergic antagonist properties. In particular, the compound has high affinity for dopamine 1, 2, 3 and 4 receptors, and for serotonin 2A, 2C, IA, 6 and 7 receptors as well as noradrenaline-al and 2 receptor types. A method for preparing asenapine is disclosed in e.g. US 4,145,434.

Asenapine (in form of its maleate salt) has been launched in the US for the treatment of acute schizophrenia and bipolar disorder. The compound has also been approved in the EU, and subsequently launched in Denmark, for the treatment of manic episodes in bipolar I disorders. The maleate salt of asenapine can be prepared by adding one molar equivalent of an ethanolic solution of maleic acid to an ethanolic solution of asenapine. For further purification, the thus obtained asenapine maleate, can be recrystallized from ethanol (see e.g. Scheme 1 in WO 2006/106135).

It is known in the art that asenapine shows low bioavailability when taken up via the gastrointestinal tract, and that it has substantially less cardiovascular side effects on sublingual or buccal administration (see WO 95/23600). In order to address these issues, WO 95/23600 disclosed a pharmaceutical composition comprising asenapine maleate for sublingual or buccal administration.

Generally speaking, the particle size of the drug substance influences several properties of the drug product, such as manufacturing, dissolution and bioavailability. For sublingual or buccal administration, the drug product should completely dissolve in the oral cavity within a short time frame. Hence, the particle size of the drug substance is very important, i.e. if drug substance particles are small it takes only short periods of time to achieve high concentrations. There was thus a need in the art to provide an oral dosage form that can readily disintegrate in the mouth.

Two different polymorphic forms of asenapine maleate are known in the art. Funke et al. (Arzneim.-Forsch./ Drug Res. 40 (1999), Nr. 5, 536-539) disclose physico-chemical properties of a first crystal form of asenapine maleate (also designated as "monoclinic form" or "form H"). EP 1 710 245 A1 and WO 2006/106135 disclose a further crystal form of asenapine maleate (designated as "orthorhombic form" or "form L"). According to the disclosure ofthe latter documents, asenapine maleate in the monoclinic form is comprised of crystalline particles over 100 $\mu$m in size and it was not possible to reproducibly obtain asenapine maleate with high polymorph purity by micronization of the monoclinic form of asenapine (i.e. by mechanically reducing the particle size of form H). In particular, it is disclosed that either form H, or form L or a mixture of both forms was obtained after micronization starting with form H (see page 2, lines 22-32 and examples 9 and 10 of WO 2006/106135)

It is known in the art that distinct physical properties of different polymorphs of the same compound can render different polymorphs more, or less, useful for a particular purpose, such as for a pharmaceutical formulation. The marketed drug product is a sublingual tablet containing the orthorhombic form of asenapine maleate (i.e. form L) and between approximately 10 % and 24 % amorphous asenapine maleate, but no monoclinic form of asenapine maleate (i.e. form H). Although it was found that form H is better soluble compared to form L, form L was used in the marketed sublingual tablet since small particle sizes of form H in a pure polymorphic form could not be reproducibly obtained by micronization (see above). Hence, there is an ongoing need in the art to provide a sublingual or buccal dosage form comprising microcrystalline asenapine maleate that shows an improved bioavailability compared to the marketed drug product containing the polymorphic form L.

EP 1 710 245 and WO 2006/106135 further disclose the provision of a pharmaceutical composition comprising the orthorhombic form of asenapine, which composition is produced via lyophilization. However, a significant drawback of this lyophilization process is that it leads to an expensive production, in particular due to the long duration of each freeze drying cycle (normally 24 to 48 hours) and the necessity to use sophisticated equipment for carrying out the process. Moreover, freeze-dried compositions are generally not stable enough to be easily handled by the modem high-speed packaging machines. Hence, there is an ongoing need in the art to provide a sublingual or buccal dosage form comprising asenapine maleate that does not require use of expensive equipment and can be prepared by a simple procedure in an efficient manner.

A further significant drawback of this lyophilization process is that it leads to a tablet not only containing the orthorhombic form of asenapine maleate (i.e. form L), but also approximately 10 % and 24 % amorphous asenapine maleate. However, it is often undesirable to incorporate a mixture of crystalline compound and amorphous compound in medicaments since this may be connected to stability issues. In particular, it is desirable that, if such mixtures are incorporated in a medicament, the mixing ratio be stable within a long time frame. Hence, there is also an ongoing need in the art to provide a sublingual or buccal dosage form comprising asenapine maleate that is stable on storage for a long period of time (e.g., with regard to impurity level, crystallinity grade or crystal form).

There is thus a need for sublingual or buccal dosage forms comprising microcrystalline asenapine maleate, which dosage forms avoid one or more problems of the conventional dosage forms comprising orthorhombic asenapine maleate.

## OBJECT AND SUMMARY OF THE INVENTION

The object of the present invention was to find for asenapine maleate a solid dosage form which is suitable for sublingual or buccal administration and is readily disintegrated in the saliva. The dosage form should be stable on storage for a long period of time (e.g., with regard to impurity level, crystallinity grade or crystal form). In particular, it was a further object of the present invention to provide a sublingual or buccal dosage form comprising asenapine maleate, which dosage form does not require use of expensive equipment and can be prepared by a simple procedure in an efficient manner. The dosage form should have a simple composition and should not comprise any excipients which are questionable from a toxicological point of view. Furthermore, the dosage form should not comprise a significant amount of amorphous asenapine maleate. Moreover, the dosage form should comprise a solid form of asenapine maleate that is better soluble at physiological or slightly acidic conditions compared to form L of asenapine maleate.

It is known in the art that amorphous asenapine maleate is hygroscopic and is less stable than the crystalline forms of asenapine maleate, in particular if exposed to increased humidity (see e.g. Hilfiker et al., Polymoprhism in the Pharmaceutical Industry, chapter 10, WILEY-VHC Verlag GmbH & Co. KGaA, Weinheim, 2006). The present inventors have also discovered that a freeze-dried formulation comprising form L of asenapine maleate and amorphous asenapine maleate is rather hygroscopic (and is chemically less stable than the inventive dosage form). Hygroscopic formulations, however, are difficult to handle under common pharmaceutical processing conditions and they ask for special techniques and equipment in order to avoid e.g. chemical degradation or polymorphic transformation during processing. Furthermore, hygroscopic formulations must often be packaged into special containments in order to ensure proper product quality during storage. Hence, special and expensive containments are necessary for the conventional formulations comprising asenapine in order to ensure proper product quality during storage.

It was a further object of the present invention to provide for asenapine maleate a solid dosage form that does not require special and/or expensive containments in order to ensure proper product quality during storage, in particular if stored under extensive humidity conditions. The solid form of asenapine maleate comprised by that dosage form should be less hygroscopic and should not be subject to transformation into other solid forms, in particular if stored under extensive humidity conditions. It was a further object of the present invention to reproducibly provide microcrystalline monoclinic asenapine maleate. It was a further object of the present invention to provide for asenapine maleate a solid dosage form that is less hygroscopic than the conventional freeze-dried dosage forms.

These objectives as well as others which will become apparent from the ensuing description are attained by the subject matter of the independent claims. Some of the preferred embodiments of the present invention are defined by the subject matter of the dependent claims.

Unexpectedly, a solid dosage form which meets the above requirements has been found in the form of a solid dosage form which contains monoclinic asenapine maleate in the form of microcrystalline material, which is prepared through the use of a solvent mediated transformation technique using a novel crystalline form of asenapine maleate, designated as crystalline form S of asenapine maleate (see Example 2). It has been found that said microcrystalline material of monoclinic asenapine maleate (i.e. produced by using said solvent mediated transformation technique) contains 5% or less of another crystalline form, or even no detectable amount of another crystalline form (in particular, form L of asenapine maleate), respectively.

Accordingly, an embodiment of the present invention relates to a compressed pharmaceutical dosage form intended for sublingual or buccal administration and capable of being rapidly disintegrated, the compressed pharmaceutical dosage form containing monoclinic asenapine maleate in a crystalline form and at least one further excipient, wherein the particle size distribution of monoclinic asenapine maleate is characterized by a d95 of less than 75 11m, and wherein the crystalline form contains less than 5 % w/w of asenapine maleate in the crystalline orthorhombic form, and optionally contains no detectable amount of asenapine maleate in the crystalline orthorhombic form.

A further embodiment of the present invention relates to a method for the preparation of the compressed pharmaceutical dosage form as described herein or according to any one of claims 1 to 10, the method comprising:

a) mixing a crystalline form of monoclinic asenapine maleate with at least one further excipient, wherein the particle size distribution of monoclinic asenapine maleate is characterized by a d95 of less than 75 μm, and wherein the crystalline form contains less than 5 % w/w of asenapine maleate in the crystalline orthorhombic form, and optionally contains no detectable amount of asenapine maleate in the crystalline orthorhombic form; and
b) compressing the mixture obtained from a) into a compressed pharmaceutical dosage form.

A further embodiment of the present invention relates to a compressed pharmaceutical dosage form intended for sublingual or buccal administration and capable of being rapidly disintegrated, which compressed dosage form is obtainable by a method comprising:

a) mixing a crystalline form of monoclinic asenapine maleate with at least one further excipient, wherein the particle size distribution of monoclinic asenapine maleate is characterized by a d95 of less than 75 μm, and wherein the crystalline form contains less than 5 % w/w of asenapine maleate in the crystalline orthorhombic form, and optionally contains no detectable amount of asenapine maleate in the crystalline orthorhombic form; and
b) compressing the mixture obtained from a) into a compressed pharmaceutical dosage form.

A further embodiment of the present invention relates to a container comprising a compressed pharmaceutical dosage form as described herein or according to any of claims 1 to 10, wherein the container is prepared from a material having a permeability for water vapor as measured according to DIN 53122 of from 1,0 g * m$^{-2}$ * d$^{-1}$ to 5000 g * m$^{-2}$ * d$^{-1}$.

**FIGURE LEGENDS**

The accompanying drawings, which are incorporated and form part of the specification, merely illustrate certain embodiments of the present invention. They are meant to serve to explain specific modes of the present invention to those skilled in the art. In the drawings:

**Figure 1:** XRPD pattern of monoclinic asenapine maleate in the form of crystalline material having a particle size distribution characterized by a d95 of 34.9 11m obtained from example 2c).

**Figure 2:** XRPD pattern of monoclinic asenapine maleate in the form of crystalline material having a particle size distribution characterized by a d95 of 10.0 μm obtained from example 2d).

**Figure 3:** XRPD pattern of monoclinic asenapine maleate in the form of crystalline material having a particle size distribution characterized by a d95 of 34.9 11m obtained from example 2c) to which 5% (a) or 10% (b) ofthe orthorhombic asenapine maleate have been added.

**Figure 4:** XRPD pattern of the dosage form commercially available under the trade name Sycrest® (based on the dosage form according to example 16 of W02006/106135); bottom diffractogram: 5 mg of the dosage form prior to determination of hygroscopicity (80% rH, 25 °C, 24h); top diffractogram: 5 mg of the dosage form after determination of hygroscopicity (80% rH, 25 °C, 24h).

**DEFINITIONS**

The present invention illustratively described in the following may suitably be practiced in the absence of any element or elements, limitation or limitations, not specifically disclosed herein.
The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes. Where the term "comprising" is used in the present description and claims, it does not exclude other elements or steps. For the purposes of the present invention, the term "consisting of is considered to be a preferred embodiment of the term "comprising of'. If hereinafter a group is defined to comprise at least a certain number of embodiments, this is also to be understood to disclose a group which optionally consists only of these embodiments.
Where an indefinite or definite article is used when referring to a singular noun e.g. "a" or "an", "the", this includes a plural of that noun unless something else is specifically stated.
The term "about" in the context of the present invention denotes an interval of accuracy that the person skilled in the art will understand to still ensure the technical effect of the feature in question. The term typically indicates deviation from the indicated numerical value of ± 10%, and preferably ± 5%.
Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Monoclinic form of asenapine maleate: the crystalline form of asenapine maleate named "form H" or "monoclinic form" as characterized in WO2006/1061 by the two most characteristic peaks at 9.6° and 26.8° 2-Theta. According to page 4, lines 17-20 of WO2006/106135, the monoclinic form of asenapine maleate can be further characterized by peaks at 2-theta of 9.6°, 20.4°, 22.0°, 23.4°, 25.2° and 26.8°.

Orthorhombic form of asenapine maleate: the crystalline form of asenapine maleate named "form L" or "orthorhombic form" as characterized in WO2006/1061 by the two most characteristic peaks at 10.5° and 15.7° 2-Theta. According to page 4, lines 22-25 of WO2006/106135, the orthorhombic form of asenapine maleate can be further characterized by peaks at 2-theta of 10.5°, 15.7°, 18.3°, 19.0°, 22.2°, 23.2° and 27.5°.

The term "capable of being rapidly disintegrated" as used herein denotes a dosage form having a disintegration time of less than 1 min, preferably of less than 30 seconds (measured according to item 2.9.1 Test A of the European Pharmacopoeia).

The term "microcrystalline" as used herein denotes that the dosage form comprises crystalline particles having a size distribution characterized by a mean particle size of less than about 50 $\mu$m and/or a d95 of 75 $\mu$m or less. In the context of the present invention, the term "d90" (or d(0.90)) with regard to particle size distribution means that 90% of the particles (based on volume) are smaller than or equal to the indicated size (measured according to item 2.9.31. of the European Pharmacopoeia). In the context of the present invention, the term "d95" (or d(0.95)) with regard to particle size distribution means that 95% of the particles (based on volume) are smaller than or equal to the indicated size (measured according to item 2.9.31. of the European Pharmacopoeia). The method disclosed in item 2.9.31. of the European Pharmacopoeia is based on the ISO standards 13320-1(1999) and 9276-1(1998).

Methods of recovering the crystalline material from compressed dosage forms are known to the person skilled in the art (e.g., by using common extraction techniques).

The term "dosage form intended for sublingual or buccal administration" as used herein denotes a pharmaceutical composition (such as a tablet) which is for insertion into the buccal pouch or for placement under the tongue of a subject, such as a human.

The term "compressed dosage form" as used herein denotes solid preparations (e.g., tablets) each containing a single dose of one or more active substances. Examples of compressed dosage forms include uncoated tablets; coated tablets; effervescent tablets; soluble tablets; dispersible tablets; orodispersible tablets; and tablets for use in the mouth. Such compressed dosage forms can be prepared by compressing uniform volumes of particles or particle aggregates produced by granulation methods. The particles consist of one or more active substances with or without excipients such as diluents, binders, disintegrating agents, glidants, lubricants, substances capable of modifying the behaviour of the preparation in the digestive tract, colouring matter authorised by the competent authority and flavouring substances. In the manufacture of such tablets, means are taken to ensure that they possess a suitable mechanical strength to avoid crumbling or breaking on handling or subsequent processing. The process of providing compressed dosage forms is well known to the skilled person.

The term "micronized" (or "micronization") as used herein denotes the process of mechanically reducing the average diameter of a solid material's particles. Traditional micronization techniques are based on friction to reduce particle size. Such methods include milling, bashing and grinding. A typical industrial mill is composed of a cylindrical metallic drum that usually contains steel spheres. As the drum rotates the spheres inside collide with the particles of the solid, thus crushing them towards smaller diameters. In the case of grinding, the solid particles are formed when the grinding units of the device rub against each other while particles of the solid are trapped in between. Methods like crushing and cutting are also used for reducing particle diameter, but produce more coarse particles compared to the two previous techniques (and are therefore the early stages of the micronization process). Crushing employs hammer-like tools to break the solid into smaller particles by means of impact. Cutting uses sharp blades to cut the coarse solid pieces into smaller ones. All these techniques are well known to the skilled person.

In the context of the present invention the following abbreviations have the indicated meaning, unless explicitly stated otherwise:

| | |
|---|---|
| XRPD: | Powder X-ray diffraction |
| r.h. or RH: | relative humidity |
| r.t.: | room temperature |
| DSC: | Differential scanning calorimetry |

Further definitions of term will be given in the following in the context of which the terms are used.

**DETAILED DESCRIPTION OF THE INVENTION**

The present invention relates to rapidly disintegrating oral dosage forms of asenapine maleate intended for sublingual or buccal administration, in particular tablets, and to methods of producing such oral dosage forms. The oral dosage forms disclosed by the present invention can be manufactured using conventional manufacturing equipment. It is thus possible to produce them with a beneficial cost structure.

In the course of crystallization experiments carried out on asenapine maleate (including solutions with ethanol, isopropanol, n-butanol, methylisobutylketon, tetrahydrofuran, ethylacetate or isopropylacetate as solvent) it has been found that the maleate salt normally crystallizes as form H, whereas some evaporation and cooling crystallization experiments led to amorphous samples. However, it has been unexpectedly found that a crystalline form of asenapine maleate, designated as crystalline form S of asenapine maleate can be prepared by crystallization of asenapine maleate in a process that comprises:

a) dissolving asenapine maleate in methanol or dissolving the free base of asenapine maleate together with maleic acid in methanol,
b) adding an ether (such as methyl tert-butyl ether or diisopropyl ether) at room temperature as antisolvent,
c) optionally adding small amounts of form S of asenapine maleate as seeds,
d) cooling the solution to 0°C to 5°C, and
e) isolating the crystals by filtration.

An exemplary process of producing crystalline form S of asenapine maleate is described in Example 2.

It has further been found that the crystalline form S of asenapine maleate is particularly suitable for preparing a monoclinic form H of asenapine maleate via a solvent-mediated process. Said solvent-mediated process comprises:

a) providing a suspension of asenapine maleate form S and isopropanol,
b) allowing the asenapine maleate form S to transform to monoclinic form H at a temperature of at most 50°C (e.g., 3-4 hours),
c) optionally isolating the crystalline form H of asenapine maleate as obtained in b) by filtration, and
d) optionally washing the crystalline form H of asenapine maleate as obtained in b) or as isolated in c) with isopropanol, and drying the crystalline form H of asenapine maleate in vacuum.

An exemplary process of producing crystalline form H of asenapine maleate via the solvent-mediated process is described in Example 2.

Isopropanol is the preferred solvent for the transformation of form S to the monoclinic form H because monoclinic form H is stable in this solvent and does not convert to the orthorhombic form L. A suspension of form H in isopropanol was found to be stable for two weeks at 27°C and for four days at 50°C. Unexpectedly, the crystal form of asenapine maleate provided by said solvent mediated transformation technique contains 5% or less of another crystalline form (particularly of form L), or even no detectable amount of another crystalline form (particularly of form L).

Another advantage of this process compared to existent methods is the preparation of the drug substance as monoclinic form H in the form of small crystals. Known crystalline asenapine maleate form H is typically comprised of crystalline particles sizes of more than 100 11m (e.g., see page 1, lines 24-27 of WO 2006/106135). To reduce the particle size of the crystals a micronization step would have to be applied, but, according to the disclosure on page 2, lines 22-32 of WO 2006/106135, it was not possible to reproducibly obtain small crystals of the monoclinic form of asenapine with high polymorph purity by micronization. As described in the document either the monoclinic form, or the orthorhombic form or a mixture of polymorphs was obtained after micronization starting with the monoclinic form comprised of large particles sizes. Even when the starting material was taken from the same batch of the monoclinic form of asenapine maleate, the document discloses that micronization resulted in product that was not reproducible. Without wishing to be bound by any theory, it is believed that it was not possible to provide pure form H and that traces of other polymorphic forms and/or amorphous asenapine might have triggered the conversion into other solid forms during micronization.

It was now found that the above-described solvent-mediated process via form S of asenapine maleate facilitates the preparation of microcrystalline monoclinic form H. Unexpectedly, it was also possible to reproducibly obtain small crystals of this monoclinic form of asenapine with high polymorph purity by micronization (see Example 2). Without wishing to be bound by any theory, it is believed that the high degree of polymorphic purity for the monoclinic form of asenapine maleate obtainable by the above-described solvent-mediated process via form S could allow the further micronization.

A solid dosage form which meets the above-mentioned objectives has been found in the form of a solid dosage form which comprises microcrystalline monoclinic asenapine maleate that is prepared through the use of said solvent mediated transformation technique via said crystalline form S of asenapine maleate. Accordingly, an embodiment ofthe present invention relates to a compressed pharmaceutical dosage form intended for sublingual or buccal administration and capable of being rapidly disintegrated, the compressed pharmaceutical dosage form containing monoclinic asenapine maleate in a microcrystalline form and at least one further excipient, and wherein the microcrystalline form contains less than 5 % w/w of asenapine maleate in the crystalline orthorhombic form, optionally contains no detectable amount of asenapine maleate in the crystalline orthorhombic form.

In particular, this embodiment of the present invention relates to a compressed pharmaceutical dosage form intended for sublingual or buccal administration and capable of being rapidly disintegrated, the compressed pharmaceutical dosage form containing monoclinic asenapine maleate in a crystalline form and at least one further excipient, wherein the particle size distribution of monoclinic asenapine maleate is characterized by a d95 of less than 75 $\mu$m, and wherein the crystalline form contains less than 5 % w/w of asenapine maleate in the crystalline orthorhombic form, and optionally contains no detectable amount of asenapine maleate in the crystalline orthorhombic form.

It has been found that the inventive dosage form offers a series of advantages compared to traditional dosage forms:

- The inventive dosage form is less hygroscopic than the conventional freeze-dried dosage forms.
- The inventive dosage form is chemically more stable than the known dosage forms comprising asenapine maleate (i.e. form L) and a certain amount of amorphous asenapine maleate.
- The microcrystalline monoclinic asenapine maleate comprised by the inventive dosage form shows a constant crystallinity grade during storage, even if kept under high humidity conditions.
- The inventive dosage form does not require special and/or expensive containments in order to ensure proper product quality during storage, in particular if stored under extensive humidity conditions.
- The microcrystalline monoclinic asenapine maleate comprised by the inventive dosage form is better soluble under physiological and/or slightly acidic conditions compared to orthorhombic asenapine maleate. In consequence, the inventive dosage form can show a better bioavailability compared to the convential dosage forms comprising orthorhombic asenapine maleate.
- For sublingual or buccal administration a drug substance with a small particle size is desired. Unexpectedly, the microcrystalline monoclinic asenapine maleate comprised by the inventive dosage form can be provided with small particle size distributions. In consequence, the inventive dosage form can rapidly dissolve in the saliva.

Asenapine contains two chiral centres and thus exists in the form of optical isomers (i.e. enantiomers). The present invention relates to all isomers of asenapine and its pharmaceutically acceptable salts. Also all tautomeric and optical forms, as well as mixtures thereof, also racemic mixtures, are included. In an optional embodiment, asenapine maleate is present in the oral dosage form in an amount of from 2 % to 50 % (w/w), from 5 % to 40 % (w/w), from 5 % to 30 % (w/w) or from 5 % to 20 % (w/w). In another optional embodiment, asenapine maleate is present in the oral dosage form in an amount of from 10 % to 40 % (w/w), from 10 % to 30 % (w/w), from 10 % to 25 % (w/w) or from 15 % to 20 % (w/w).

Optionally, the asenapine maleate in the crystalline monoclinic form has a particle size distribution characterized by a d95 of at most 50 $\mu$m, optionally at most 30 $\mu$m. In an optional embodiment, the asenapine maleate in the crystalline monoclinic form has a particle size distribution characterized by a d90 of at most 50 $\mu$m, optionally at most 30 $\mu$m. In another optional embodiment, the asenapine maleate in the crystalline monoclinic form has a mean particle size of less than about 25 $\mu$m, more preferably of less than about 20 $\mu$m, and most preferably of less than about 15 $\mu$m. In consequence, the inventive dosage form can rapidly dissolve in the saliva. Optionally, the asenapine maleate in the crystalline monoclinic form has a particle size distribution characterized by a d95 of between 50 $\mu$m and 1 $\mu$m, between 30 $\mu$m and 1 $\mu$m, or between 20 $\mu$m and 1 $\mu$m.

The combination of a particle size distribution characterized by a d95 of at most 50 $\mu$m and comprising less than 5% w/w of the orthorhombic crystalline form of asenapine maleate in relation to the total amount of asenapine maleate, and most preferably no detectable amount of the orthorhombic crystalline form of asenapine maleate; the combination of a particle size distribution characterized by a d95 of at most 30 $\mu$m and comprising less than 5% w/w of the orthorhombic crystalline form of asenapine maleate in relation to the total amount of asenapine maleate, and most preferably no detectable amount of the orthorhombic crystalline form of asenapine maleate; and the combination of a particle size distribution characterized by a d95 of at most 25 $\mu$m and comprising less than 5% w/w of the orthorhombic crystalline form of asenapine maleate in relation to the total amount of asenapine maleate, and most preferably no detectable amount of the orthorhombic crystalline form of asenapine maleate, are particularly preferred embodiments.

The present invention relates to a compressed dosage form intended for sublingual or buccal administration which is capable of being rapidly disintegrated, for example when put into the oral cavity, in particular under the tongue. The inventive oral dosage forms are compressed, yet they are still capable of disintegrating rapidly. In a further optional embodiment the compressed dosage forms of the present invention show a disintegration time from 5s to 50s, from 5s to 40s, or from 5s to 30s. In a further optional embodiment the compressed dosage forms of the present invention show a disintegration time from 5s to 30s. In this context, the term "disintegration time" refers to a measurement in pure water at 37°C according to the method described in Eur. Pharm. chapter 2.9.1 Test A.

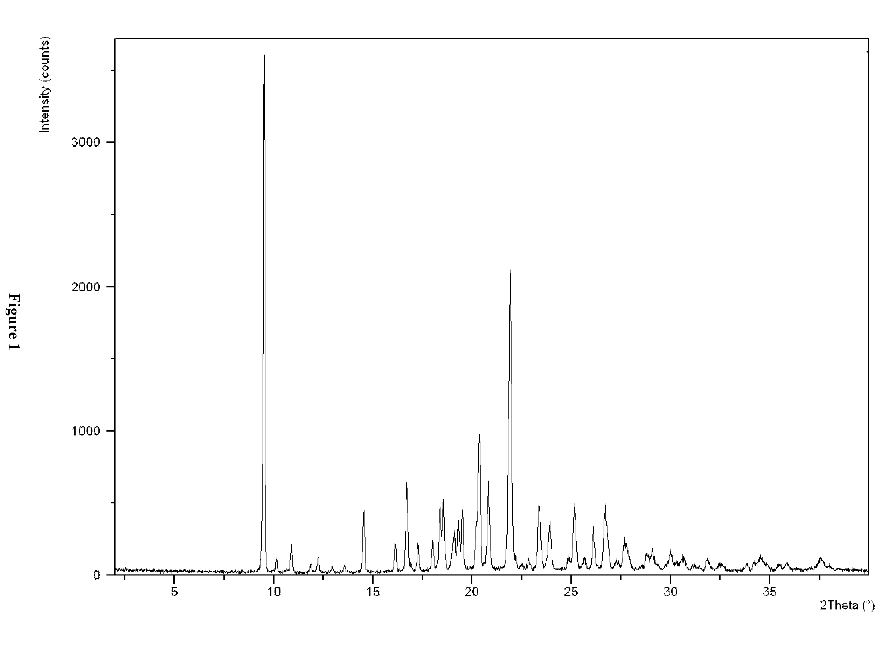

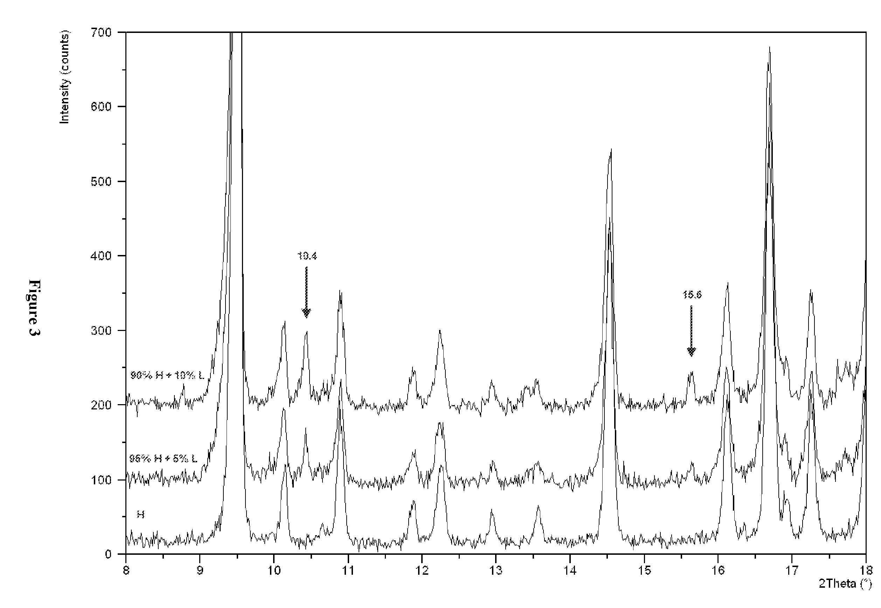

The present invention relates to dosage forms, wherein more than 95 % of asenapine maleate is stably present as asenapine maleate in monoclinic form, and more preferably wherein asenapine maleate in monoclinic form is the only detectable crystalline form of asenapine maleate. The absence or presence of other polymorphic forms of asenapine maleate, such as the orthorhombic form, can be tested by comparing an XRPD taken of any crystalline asenapine maleate with the XRPD of the monoclinic form as obtained e.g. from example 2 and shown in Figure 1, which for this comparison is to be taken as an XRPD of 100% monoclinic form, respectively. For instance, Figure 3 depicts a XRPD pattern of monoclinic asenapine maleate in the form of crystalline material having a particle size distribution characterized by a d95 of 34.9 $\mu$m obtained from example 2c) to which 5% (a) or 10% (b) of the orthorhombic asenapine maleate have been added.

The term "stably present" as used herein denotes that even after storage of the dosage form for 180 days in conventional containers (blisters) (e.g., 25°C, 60% r.h.; or 40°C, 75% r.h.), and preferably even after storage for 3 years, the monoclinic form of asenapine maleate initially comprised in the pharmaceutical dosage form is still present as asenapine maleate in the monoclinic form after storage for the indicated period.

According to an optional embodiment the asenapine maleate comprised by the compressed pharmaceutical dosage form is characterized by an X-ray powder diffraction pattern not comprising a peak at the 2- theta angle of 15.7 $\pm$ 0.2°, optionally characterized by an X-ray powder diffraction pattern substantially in accordance with figure 1. Preferably, also the compressed pharmaceutical dosage form is characterized by an X-ray powder diffraction pattern not comprising a peak at the 2- theta angle of 15.7 $\pm$ 0.2°.

The main characteristics of diffraction line profiles are 28 position, peak height, peak area and shape (characterised by, for example, peak width or asymmetry, analytical function, empirical representation). In addition to the diffraction peaks, an X-ray diffraction experiment also generates a more-or-less uniform background, upon which the peaks are superimposed. Besides specimen preparation, other factors contribute to the background, for instance the sample holder, diffuse scattering from air and equipment, other instrumental parameters such as detector noise, general radiation from the X-ray tube, etc. The peak-to-background ratio can be increased by minimising background and by choosing prolonged exposure times. In the context of the present invention, the term "peak" denotes a particular 28 position, wherein the signal-to-noise ratio (calculated according to item 2.2.46 of the European Pharmacopoeia) is greater than 3/1.

Suitable excipients for use in the present invention include preservatives, fillers, sweeteners, flavoring agents, coloring agents, disintegrating agents, glidants, antiadherents, lubricants and mixtures thereof. Other excipients known in the field of pharmaceutical compositions may also be used. Furthermore, the dosage form may comprise a combination of two or more excipients also within one of the members of the above-mentioned group.

According to an optional embodiment, the at least one further excipient is a disintegrant. According to an optional embodiment the inventive dosage form comprises 1 % to 40.0 % (w/w), 2.0 % to 20.0 % (w/w), 3.0 % to 15.0 % (w/w) or 5 % to 10 % (w/w) of a disintegrant. According to a further optional embodiment the inventive dosage form comprises 2.0 % to 10.0 % (w/w) or 2.5 % to 5.0 % (w/w) of a disintegrant. Optionally, the disintegrant is selected from the group consisting of cross-linked carboxymethylcellulose sodium, cross-linked polyvinylpyrrolidone, pregelatinized starch, sodium carboxymethyl cellulose, calcium carboxymethyl cellulose, sodium alginate, sodium starch glycolate, crospovidone, croscarmellose sodium and low substituted hydroxyl propyl cellulose. Optionally, the disintegrant is cross-linked polyvinylpyrrolidone (commercially available e.g. under the trade name Kollidon CL). Optionally, the disintegrant is cross-linked low substituted hydroxyl propyl cellulose.

In an optional embodiment the inventive dosage form comprises an insoluble or poorly soluble cross-linked polymeric disintegrant. Such disintegrating agents are optionally selected from sodium starch glycolate, crospovidone, croscarmellose sodium and low substituted hydroxy propyl cellulose, with sodium starch glycolate being particularly preferred. It is optionally added to a final concentration of less than 10 % (w/w), particularly below 5 % (w/w). Sodium starch glycolate is commercially available e.g. under the trade name Primoj el.

Optionally, the disintegrant is cross-linked carboxymethylcellulose (also known as Croscarmellose). It is optionally added to a final concentration of between about 0.1 % and 10.0 % (w/w), preferably of between about 0.5 % and 5.0 % (w/w). Optionally, the disintegrant is Crospovidone. It is optionally added to a final concentration of between about 0.5 % and 6.0 % (w/w), preferably of between about 1.0 % and 3.0 % (w/w). Optionally, the disintegrant is L-HPC. It is optionally added to a final concentration of between about 0.5 % and 10.0 % (w/w), preferably of between about 1.0 % and 5.0 % (w/w). Optionally, the disintegrant is sodium starch glycolate. It is optionally added to a final concentration of between about 0.5 % and 8.0 % (w/w), preferably of between about 1.0 % and 4.0 % (w/w).

In a further optional embodiment the further excipient is a filler present in an amount of from 15 % to 90 % (w/w). Particularly suitable fillers include lactose, mannitol, sorbitol, xylitol, powdered cellulose, microcrystalline cellulose, calcium carbonate, dibasic calcium phosphate and mixtures thereof. In an optional embodiment the filler is a cellulose-type filler. It can be selected from powdered cellulose and microcrystalline cellulose. Cellulose type filler can be used alone or in combination with (a) further soluble filler(s). The compressed oral dosage forms of the present invention optionally contain microcrystalline cellulose as filler used alone or in combination with any one of the above-mentioned fillers. In a further optional embodiment filler is a mixture of microcrystalline cellulose and mannitol. Although other fillers such as calcium carbonate, dibasic calcium phosphate etc can be added, microcrystalline cellulose is most preferred. Microcrystalline cellulose in the range of from 5 % to 50 % (w/w) is found suitable. Optionally, in those embodiments where microcrystalline cellulose is not used as the sole filler, the microcrystalline cellulose is present in an amount of from 5 % to 30 % (w/w) of total filler, preferably of from 10 % to 20 % (w/w) of total filler. Microcrystalline cellulose has good compressibility characteristics and tablets produced thereof are of sufficient hardness and along with asenapine maleate, rapidly disintegrating tablets of sufficient hardness are successfully produced which in mouth can disintegrate within 60 seconds, and optionally within 30 seconds. Optionally, the inventive dosage form comprises microcrystalline cellulose as filler (e.g., commercially available under the trade name Avicel).

Amongst soluble fillers are lactose, sorbitol, xylitol and mannitol. Optionally, microcrystalline cellulose is present in the inventive dosage form in an amount of from 10 % to 20 % (w/w) of total filler, and mannitol is present in the inventive dosage form in an amount of from 80 % to 90 % (w/w) of total filler. According to an optional embodiment the inventive dosage form comprises 40.0 % to 90.0 % (w/w), 60.0 % to 90.0 % (w/w), 70.0 % to 90.0 % (w/w) or 75 % to 85 % (w/w) of total filler (i.e. one or more fillers). Optionally, the inventive dosage form comprises a mixture of microcrystalline cellulose and mannitol (commercially available e.g. under the trade name Pearlitol 200SD) as filler. Optionally, the inventive dosage form comprises mannitol (commercially available e.g. under the trade name Pearlitol 200SD) as filler. Optionally, the inventive dosage form comprises lactose (commercially available e.g. under the trade name Tablettose 70) as filler. Mannitol by virtue of its negative heat of dissolution, pleasant taste and mouth feel is a good filler for mouth dissolve tablets. Lactose combines flowability of coarse crystals and good compressibility of a fine milled lactose.

According to an optional embodiment the further excipient is a lubricant. Particularly suitable lubricants include calcium stearate, magnesium stearate, sodium stearyl fumarate, stearic acid in particular stearic acid and/or its salts. It is preferred that the lubricant be present in an amount from 0.5 % to 5.0 % (w/w).

Faculatively, talc, colloidal silicon dioxide, magnesium or calcium stearate can be added to improve flow properties of the blend. According to an optional embodiment the further excipient is a glidant, optionally being selected from colloidal silicon dioxide and talc. Preferably, colloidal silicone dioxide and/or talc is present in an amount of from 0.1 % to 3.0 % (w/w).

According to an optional embodiment the further excipient is a sweetener, optionally being selected from powdered sugar, saccharine, aspartame, cyclamates, in particular selected from the group consisting of cyclamate or salts thereof; saccharin or salts thereof; and aspartame. According to an optional embodiment the further excipient is a flavouring agent, optionally being selected from vanilla, banana, strawberry, caramel, orange, lemon and cherry, with strawberry being particularly preferred.

The compressed dosage forms of the invention have an improved crushing strength when compared to freeze-dried formulations of the prior art. Thus, the present invention, in an optional embodiment, relates to compressed dosage forms having a crushing strength (according to item 2.9.8 of the European Pharmacopoeia) of from 5 N to 50 N, preferably of from 7 N to 40 N, more preferably from 7 N to 30 N, most preferably from 7 N to 20 N.

Optionally, the compressed oral dosage forms of the present invention do not require binder in the formulation.

The compressed oral dosage forms of the present invention can be formulated by simple blending followed by direct compression. The dosage forms produced by this technique have sufficient hardness to make it suitable to pack on high speed packing machines without requiring any special care and precaution for handling as well as packing of the dosage forms. The dosage forms maintain the sufficient hardness throughout their shelf life. This is a great advantage of the present dosage form over freeze dried tablet formulations which lack hardness and require special care in handling.

The dosage form according to the invention can be in any suitable form such as a tablet, a capsule or a granulate. Preferably, it takes the form of a tablet. It has surprisingly been shown that the dosage form according to the invention overcomes the problems associated with the conventional asenapine formulations produced by freeze-drying. The inventive dosage form not only disintegrates very quickly in the oral cavity (thus resulting in good patient compliance), but does further not require special and/or expensive containments in order to ensure proper product quality during storage, in particular if stored under extensive humidity conditions. Additionally, the preparation of the inventive dosage form does not require use of expensive equipment and the dosage form can be prepared by a simple procedure in an efficient manner.

Surprisingly, the dosage form of the present invention is less hygroscopic than the conventional freeze-dried dosage forms comprising orthorhombic asenapine maleate. In particular, the inventive dosage form only takes up about 0.9 w% water from about 80 % relative humidity at about 25 °C (see example 9), whereas the dosage form of example 16 of WO 2006/106135 takes up about 4.3 w% water from about 80 % relative humidity at about 25 °C (as measured after 24 hours according to item 5.11. of the European Pharmacopoeia).

According to an optional embodiment, the compressed pharmaceutical dosage form is not hygroscopic (increase in mass is less than 0.2 %) or only slightly hygroscopic (increase in mass is less than 2 % and equal to or greater than 0.2 %) as measured according to item 5.11. of the European Pharmacopoeia. According to another optional embodiment, the increase in mass of the inventive dosage form due to uptake of water is less than 2.0 % w/w, preferably less than 1.5 % w/w, and more preferably less than 1.0 % w/w after storage for 24 hours at 25 °C and 80 % relative humidity (as measured according to item 5.11. of the European Pharmacopoeia).

According to an optional embodiment, the inventive dosage form takes up less than 5.0 w%, preferably less than 4.0 w%, more preferably less than 3.0 w%, and most preferably less than 2.0 w% water from about 60 % $\pm$ 5 % relative humidity at about 25 °C =L 2 °C (measured after 4 weeks e.g. according to item 5.11. of the European Pharmacopoeia). According to an optional embodiment, the inventive dosage form takes up less than 5.0 w%, preferably less than 4.0 w%, more preferably less than 3.0 w%, and most preferably less than 2.0 w% water from about 75 % $\pm$ 5 % relative humidity at about 40 °C $\pm$ 2 °C (measured after 4 weeks e.g. according to item 5.11. of the European Pharmacopoeia)). According to an optional embodiment, the inventive dosage form takes up less than 5.0 w%, preferably less than 4.0 w%, more preferably less than 3.0 w%, and most preferably less than 2.0 w% water from about 65 % $\pm$ 5 % relative humidity at about 30 °C $\pm$ 2 °C (measured after 4 weeks e.g. according to item 5.11. of the European Pharmacopoeia)). According to an optional embodiment, the inventive dosage form takes up less than 5.0 w%, preferably less than 4.0 w%, more preferably less than 3.0 w%, and most preferably less than 2.0 w% water from about 75 % $\pm$ 5 % relative humidity at about 30 °C $\pm$ 2 °C (measured after 4 weeks e.g. according to item 5.11. of the European Pharmacopoeia)).

It has further been found that the dosage form of the present invention is more stable with regard to its crystallinity grade than the conventional freeze-dried dosage forms comprising orthorhombic and amorphous asenapine maleate, in particular if stored under extensive humidity conditions. The crystallinity grade of a sample can be determined by an XRPD as described in item 2.9.33. ofthe European Pharmacopoeia. In particular, it has been found that after exposing the dosage form to about 80 % relative humidity and about 25 °C for 24 hours, the dosage form of example 16 of WO 2006/106135 comprising orthorhombic and amorphous asenapine maleate showed a significant increase of its crystallinity grade (see example 10). In contrast to the conventional freeze-dried dosage forms comprising orthorhombic and amorphous asenapine maleate, the dosage form ofthe present invention contains monoclinic asenapine maleate in crystalline form.

It has further been found that the dosage form of the present invention is chemically more stable than the conventional freeze-dried dosage forms comprising orthorhombic asenapine maleate, in particular if stored under extensive humidity conditions. In particular, it has been found that the inventive dosage form comprising microcrystalline monoclinic asenapine maleate shows significantly less degradation products than the conventional freeze-dried dosage forms after long-term storage. After storage of the dosage forms (in Alu blisters) for 4 weeks (60 °C, 75 % rh), the inventive dosage form showed an increase of degradation products (i.e. impurities [%]) of less than 0.5 %, whereas the dosage form of example 16 of WO 2006/106135 showed an increase of degradation products (i.e. impurities [%]) of about 1.5 % (see example 11).

Hence, one advantage of the dosage form of the present invention is that it is less hygroscopic than the conventional dosage forms comprising orthorhombic asenapine maleate. A further advantage of the dosage form of the present invention is that it is more stable with regard to its crystallinity grade than the conventional freeze-dried dosage forms comprising orthorhombic and amorphous asenapine maleate, in particular if stored under extensive humidity conditions. A further advantage of the dosage form of the present invention is that it is chemically more stable than the conventional freeze-dried dosage forms comprising orthorhombic and amorphous asenapine maleate, in particular if stored under extensive humidity conditions.

A further advantage of the dosage form of the present invention is that it is mechnically more stable than the conventional freeze-dried dosage forms so that conventional push-through-pack (PTP) blisters can be used. In contrast, the conventional freeze-dried dosage forms are typically made of either very porous and soft- moulded matrices, which makes the dosage forms friable and/or brittle, which are difficult to handle, often requiring specialized peel-off blister packaging.

Thus, the dosage form of the present invention comprising monoclinic asenapine maleate enables the skilled person to package or fill said dosage forms into inexpensive containers or blisters. The present invention therefore also relates to a container comprising the inventive dosage forms, in particular to a container prepared from a material having a permeability for water vapor as measured according to DIN 53 122 at a foil thickness of 50 $\mu$m of from 1.0 g $*$ m$^{-2}$ $*$ d$^{-1}$ to 5000 g $*$ m$^{-2}$ $*$ d$^{-1}$, more preferably of from 5 g $*$ m$^{-2}$ $*$ d$^{-1}$ to 2000 g $*$ m$^{-2}$ $*$ d$^{-1}$. The present invention also relates to a container comprising a dosage form containing microcrystalline monoclinic asenapine maleate, in particular to a container prepared from a material having a permeability for water vapor as measured according to DIN 53 122 at a foil thickness of 50 $\mu$m of from 1.0 g $*$ m$^{-2}$ $*$ d$^{-1}$ to 5000 g $*$ m$^{-2}$ $*$ d$^{-1}$, more preferably of from 5 g $*$ m$^{-2}$ $*$ d$^{-1}$ to 2000 g $*$ m$^{-2}$ $*$ d$^{-1}$. Preferred examples of such an inexpensive packaging material with relatively high permeability for water vapor are polypropylene, polyvinylidenchloride, polyvinylchloride, polystyrole, polyamide, polyethylenevinylacetate, cellophane and celluloseacetate, with polypropylene, polyvinylidenchloride, polyvinylchloride being particularly preferred. The use of these materials for blistering the inventive dosage forms comprising microcrystalline monoclinic asenapine maleate becomes possible due to the low hygroscopicity and good stability of the inventive dosage form. This represents a clear economic advantage of the dosage form according to the invention.

The present invention also relates to the use of microcrystalline monoclinic asenapine maleate for the production of a pharmaceutical dosage form intended for sale in a tropical country having areas with an Af or Am climate according to the Köppen-Geiger climate classification. According to an optional embodiment, the inventive dosage form is intended for sale in a country having areas with an Af or Am climate according to the K6ppen-Geiger climate classification. According to another optional embodiment, the container comprising the disclosed dosage forms is intended for sale in a country having areas with an Af or Am climate according to the K6ppen-Gelger climate classification.

A further embodiment of the present invention is directed to a method for the preparation of the compressed pharmaceutical dosage form as described herein above, the method comprising:

a) Mixing a crystalline form of monoclinic asenapine maleate with at least one further excipient, wherein the particle size distribution of monoclinic asenapine maleate is characterized by a d95 of less than 75 $\mu$m, and wherein the crystalline form contains less than 5 % w/w of asenapine maleate in the crystalline orthorhombic form, and optionally contains no detectable amount of asenapine maleate in the crystalline orthorhombic form; and

b) Compressing the mixture obtained from a) into a compressed pharmaceutical dosage form. As an additional step, the monoclinic form of asenapine maleate can be micronized prior to a). A further embodiment of the present invention is directed to a compressed dosage form obtainable by this method.

The particle size distribution of monoclinic asenapine maleate in the inventive dosage form is characterized by a d95 of less than 75 $\mu$m. In optional embodiments of the invention, said particle size distribution of monoclinic asenapine maleate can be characterized by a d90 of less than 75 $\mu$m, a d95 of less than 50 $\mu$m, a d90 of less than 50 $\mu$m, a d95 of less than 30 $\mu$m, or a d90 of less than 30 $\mu$m.

A further embodiment of the present invention is directed to a method for the preparation of the compressed pharmaceutical dosage form as described herein above, the method comprising:

a) dissolving asenapine maleate in methanol or dissolving the free base of asenapine maleate together with maleic acid in methanol,

b) adding an ether (such as methyl tert-butyl ether or diisopropyl ether) at room temperature as antisolvent,

c) optionally adding small amounts of form S of asenapine maleate as seeds,

d) cooling the solution to 0°C to 5°C,

e) isolating the crystals (i.e. form S of asenapine maleate) by filtration,

f) providing a suspension of asenapine maleate form S and isopropanol,

g) allowing the asenapine maleate form S to transform to monoclinic form H at a temperature of at most 50°C (e.g., 3-4 hours),

h) optionally isolating crystalline form H of asenapine maleate as obtained in g) by filtration,

i) optionally washing the crystals (i.e. form H of asenapine maleate) with isopropanol and drying the crystals in vacuum,

j) optionally micronizing the crystals (i.e. form H of asenapine maleate),

k) mixing the microcrystalline monoclinic form of asenapine maleate with at least one further excipient, and

l) compressing the mixture obtained from k) into a compressed pharmaceutical dosage form. A further embodiment of the present invention is directed to a compressed dosage form obtainable by this method.

A further embodiment of the present invention is directed to a method for the preparation of the compressed pharmaceutical dosage form as described herein above, the method comprising:

a) providing a suspension of asenapine maleate form S and isopropanol,

b) allowing the asenapine maleate form S to transform to monoclinic form H at a temperature of at most 50°C (e.g., 3-4 hours),

c) optionally isolating crystalline form H of asenapine maleate as obtained in b) by filtration,

d) optionally washing the crystals (i.e. form H of asenapine maleate) with isopropanol and drying the crystals in vacuum,

e) optionally micronizing the crystals (i.e. form H of asenapine maleate),

f) mixing the microcrystalline monoclinic form of asenapine maleate with at least one further excipient, and

g) compressing the mixture obtained from f) into a compressed pharmaceutical dosage form. A further embodiment of the present invention is directed to a compressed dosage form obtainable by this method.

The step of compressing the mixture into a compressed pharmaceutical dosage form can be accomplished by any suitable compression method. For instance, a standard rotary press (pressure 1 to 20 kN, preferably 1-15 kN, more preferably 1-10 kN) may be used for the production of the inventive dosage form. Further methods and devices are known to the person skilled in the art.

The step of micronizing asenapine maleate crystals in the monoclinic form can be accomplished by any suitable micronization method. For instance, a Hosokawa jet mill 50 0 AS (diameter of mill chamber: 5 cm) can be used and asenapine maleate crystals can be added manually (about 3 g/min; mill chamber 50°, nozzle 0.8 mm; injection pressure 3.5 bar, milling pressure 3.5 bar). Further methods and devices are known to the person skilled in the art.

Thus, the orally-disintegrating composition according to the invention can be prepared by a simple production process which usually is employed for the production of e.g. conventional tablets. In contrast to the prior art compositions, the manufacturing process is not only very simple, but also does not require use of organic solvents, expensive excipients and inert cores. Nevertheless, the compositions show the desired combination of fast disintegration in the mouth of a patient.

According to an optional embodiment, the inventive dosage forms have a diameter ranging from about 5 mm to about 8 mm. According to a further optional embodiment, the weight of the inventive dosage forms ranges from about 30 mg to about 200 mg.

Another embodiment of the present invention relates to the above-described dosage form for use in the treatment of acute schizophrenia, bipolar disorder or manic episodes in bipolar I disorders, the dosage form comprising microcrystalline monoclinic asenapine maleate as active ingredient.

The specific nature of the products and compositions of the present invention (e. g. the nature of the additional components, the relative proportions of the components and the physical nature of the composition) will depend on the particular application and are known to the skilled person. While the above invention has been described with respect to some of its preferred embodiments, this is in no way to limit the scope of the invention. The person skilled in the art is clearly aware of further embodiments and alterations to the previously described embodiments that are still within the scope of the present invention.

EXAMPLES

The X-ray powder diffraction patterns (XRPD) were obtained with a PANalytical X'Pert PRO diffractometer equipped with a theta/theta coupled goniometer in transmission geometry, Cu-Kal,2 radiation (wavelength 0,15419 nm) with a focusing mirror and a solid state PIXcel detector. The patterns were recorded at a tube voltage of 40 kV, tube current of 40 mA, applying a stepsize of 0.007° 2-theta with 80s per step (255 channels) in the angular range of 2° to 40° 2-theta at ambient conditions. A typical precision of the 2-theta values is in the range of $\pm$ 0.2° 2-theta. Thus a diffraction peak that appears at 5.0° 2-theta can appear between 4.8 and 5.2° 2-theta on most X-ray diffractometers under standard conditions.

Differential scanning calorimetry (DSC) was performed on a Mettler Polymer DSC R instrument. About 1 - 2 mg sample were heated in 40 $\mu$l aluminium pans with pierced aluminium lids from room temperature to 250°C at a rate of 10°C/min. Nitrogen (purge rate 50 ml/min) was used as purge gas.

For the determination of the particle size distribution (PSD) the laser diffraction spectroscopy (LDS) method as described in WO2006/1061 on page 9 under the header "LDS method" was used. A Mastersizer 2000 (Malvern, UK-Worcestershire) equipped with a Hydro SM Small Sample Dispersion unit (Malvern, UK-Worcestershire) was used for PSD-Analysis. Further equipment used are: XS1003S analytical balance (Mettler Toledo, CH-Greifensee), SONOREX ultrasoniq bath (Bandelin, D-Berlin) and 0.22 $\mu$m and 0.45 um membrane filters used with an 25 mm glass micronalysis vacuum filter holder (Millipore, Billerica, MA, USA).

Reference example 1:

*Preparation of monoclinic Form H of asenapine maleate from the base similar to the method disclosed in WO2006/106135.*

*trans*-5-chloro-2,3,3a,12b-tetrahydro-2-methyl-1H-dibenz [2,3:6,7]oxepino[4,5-c] pyrrole (asenapine) was prepared according to Example 3 of WO2008/003460 by Ullman cyclisation of trans-N-methyl-2-(2-hydroxyphenyl)-3-(2-bromo-5-chlorphenyl)-pyrrolidine. The crude base compound was purified by chromatography using a mixture of dichloromethane and methanol (20:1 v/v) as eluent.

45.23 g of the purified base (HPLC: 97%+) were dissolved in 100 ml ethanol and the solution was added dropwise to a solution of 18.37 g maleic acid in 22.0 ml Ethanol at 60°C. After stirring at 60°C for another 15 min the solution was cooled down spontaneously to RT. At about 30°C seed crystals of form H were added and product started to precipitate immediately. Then 375 ml ethyl ether was added dropwise and the suspension was stirred at RT for 3h. The suspension was cooled down to 10 °C within 10-15 minutes and stirred for one hour at 10 °C. The solid was filtered off, washed with 20ml ethyl ether and dried at 40°C overnight to yield 53.26 g of asenapine maleate (HPLC: 99%+).

XRPD: complies to reference of monoclinic polymorph (not shown)

DSC: peak at 144.4°C

Particle size distribution: d(0.1) = 1.6, d(0.5) = 50.6, d(0.9) = 115.2 and d(0.95) = 128.2

Example 2:

*a) Preparation of seeds of crystalline form S of asenapine maleate*

500 mg asenapine maleate of reference example 1 and 1.5 ml methanol were briefly heated in a reaction tube until almost all of the asenapine maleate was dissolved. The solution was clarified by filtration and 8.5 ml methyl tert-butyl ether (MTBE) were added to the filtrate in one portion. The mixture was put in a refrigerator at 5°C without agitation for three days. The precipitated crystals were collected by filtration and then dried at room temperature in a vacuum oven to yield 285 mg of asenapine maleate in form of white needles.

*b) Preparation of crystalline form S of aseviapine maleate*

7.14 g of asenapine free base, trans-5-chloro-2,3,3a,12b-tetrahydro-2-methyl-1H-dibenz [2,3:6,7]oxepino[4,5-c] pyrrole (yellow oil, 0.025 mol), was dissolved in 30 ml ofmethanol and 2.9 g of solid maleic acid was added. The reaction mixture was stirred at room temperature until a clear solution was obtained. The solution was filtered dust free. Under strong stirring 150 ml of methyl tert-butyl ether (MTBE) was added. The reaction mixture was then cooled in an ice bath to 0 °C (ice/water mixture) and seeded with 0.1 g of of Asenapine maleate form S obtained from example 1a). After standing without agitation for 5 hours the precipitated crystals were filtered. The crystals were washed with 30 ml MTBE. The wet crystals were dried under vacuum (30 - 40 mbar) at room temperature over night to afford 8.2 g of asenapine maleate, form S.

*c) Transformation of form S of asenapine maleate to the monoclinic polymorph H (not micronized)*

38.56 g crystalline form S of asenapine maleate prepared according to the procedure described in example 1b) were suspended in 193 ml isopropanol and stirred with a standard mechanical stirrer (170 rpm) at room temperature. After 3 hours a small amount of material was removed, filtered and dried at room temperature while stirring of the bulk sample was continued. The small amount of solids isolated after 3 hours of stirring was found to correspond to the monoclinic form H of asenapine maleate as judged by XRPD analysis (see Figure 1).

The crystals from the bulk sample were collected by filtration after stirring the mixture for a total of 28 hours and dried in vacuum at room temperature over night.

Yield: 34.1 g of monoclinic form H of asenapine maleate

DSC: peak at 145.1°C

Particle size distribution: d(0.1) = 1.5, d(0.5) = 7.7, d(0.9) = 27.0 and d(0.95) = 34.9

*d) Transformation of form S of asenapine maleate to the monoclinic polymorph H (micronized)*

113 g crystalline form S of asenapine maleate prepared according to the procedure described in example 1b) were suspended in 570 ml isopropanol and stirred at room temperature for a total of 28 hours. The solid crystalline material was isolated by filtration and dried in vacuum at room temperature over night.

Yield: 101.7 g of monoclinic form H of asenapine maleate.

Particle size distribution: d(0.1) = 1.5, d(0.5) = 7.0, d(0.9) = 21.1 and d(0.95) = 25.1

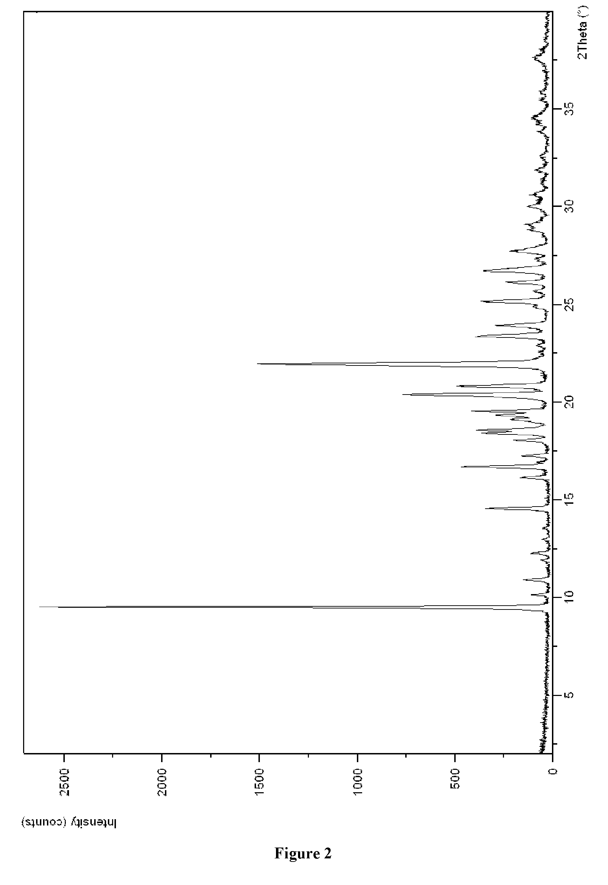

33 g of the product were micronized in a Hosokawa spiral Jet Mill 50 AS, using a micronization pressure of 3.5 bar. The micronized product was characterized as the monoclinic polymorph. Forms S and the orthorhombic form L of asenapine maleate could not be detected by XRPD, as judged by the absence of XRPD peaks at 2-theta angles (=L 0.2) of 5.5 (a characteristic peak for form S) and 10.5 and/or 15.7 (characteristic peaks of orthorhombic form L as characterized in W02006/106135) (see Figure 2). The peaks at 10.4 and/or 15.6 depicted in Figure 3 (XRPD pattern of monoclinic asenapine maleate in the form of crystalline material obtained from example 3 to which 5% (a) or 10% (b) ofthe orthorhombic asenapine maleate have been added) correspond to the two most characteristic peaks of orthorhombic asenapine maleate at 10.5° ± 0.2° and 15.7° ± 0.2° 2-Theta as characterized in W02006/106135.

Particle size distribution: d(0.1) = 1.1, d(0.5) = 3.9, d(0.9) = 8.5 and d(0.95) = 10.0

Reference example 3:

Asenapine maleate of the monoclinic form is mixed into a gelatin/mannitol matrix and dosed by weight into pre-formed pockets. The matrix is frozen within the pockets by passage through a freeze tunnel. The frozen tablets are then dried by sublimating the ice in a freeze dryer. In particular, 2000 g gelatin and 1500 g mannitol are dispersed in 45.01 kg of purified water, while stirring and heating in a vacuum mixer. After dissolution the matrix is filtered, 1406 g asenapine maleate monoclinic form is added and mixed. The mixture is dispensed using dosing pumps into pre-formed blister pockets (250 mg in each pocket). The filled pockets are frozen by passing through a liquid nitrogen freeze tunnel. The frozen tablets are dried in a freeze dryer using a pre-programmed drying cycle. Each pocket contains a pharmaceutical unit dosage comprising 7.03 mg of asenapine maleate, 10.0 mg gelatin and 7.5 mg mannitol.

Example 4:

| Composition | mg/Tbl. |
| --- | --- |
| Asenapine maleate (form H) | 7.03 |
| MCC 90 | 3.9 |
| Mannitol (Pearlitol 200SD) | 23.75 |
| Magnesium stearate | 0.26 |
| **total** | **approx. 35.0** |

Asenapine maleate according to example 2c) (monoclinic, not micronized), microcrystalline cellulose (MCC 90), mannitol and magnesium stearate in the above given amounts were mixed and compressed into tablets. MCC 90 is commercially available e.g. from FMC (under the trade name Avicel PH 102). Mannitol (Pearlitol 200SD) is commercially available e.g. from Roquette. Magnesium stearate is commercially available e.g. from Peter Greven.

Example 5:

| Composition | mg/Tbl. |
| --- | --- |
| Asenapine maleate (form H) | 7.03 |
| MCC 90 | 3.9 |
| Mannitol (Pearlitol 200SD) | 23.75 |
| Magnesium stearate | 0.26 |
| **total** | **approx. 35.0** |

Asenapine maleate according to example 2d) (monoclinic, micronized), microcrystalline cellulose (MCC 90), mannitol and magnesium stearate in the above given amounts were mixed and compressed into tablets.

Example 6:

| Composition | mg/Tbl. |
| --- | --- |
| Asenapine maleate (form H) | 7.03 |
| MCC 90 | 3.9 |
| Mannitol (Pearlitol 200SD) | 22.06 |
| Primojel | 1.75 |
| Magnesium stearate | 0.26 |
| **total** | **approx. 35.0** |

Asenapine maleate according to example 2c) (monoclinic, not micronized), microcrystalline cellulose (MCC 90), mannitol, sodium starch glycolate (Primojel) and magnesium stearate in the above given amounts were mixed and compressed into tablets. Optionally, the asenapine maleate can be micronized prior to producing the tablets (see example 2d)).

Example 7:

| Composition | mg/Tbl. |
|---|---|
| Asenapine maleate (form H) | 7.03 |
| Polyvinylpyrrolidone | 3.0 |
| Mannitol | 80.0 |
| **total** | **approx. 90.0** |

Asenapine maleate according to example 2c) (monoclinic, not micronized), polyvinylpyrrolidone (such as Kollidon 25) and mannitol in the above given amounts are mixed and compressed into tablets. Optionally, the asenapine maleate can be micronized prior to producing the tablets (see example 2d)).

Example 8:

| Composition | mg/Tbl. |
|---|---|
| Asenapine maleate (form H) | 7.03 |
| Kollidon 25 | 3.0 |
| Tablettose 70 | 80.0 |
| **total** | **approx. 90.0** |

Asenapine maleate according to example 2c) (monoclinic, not micronized), polyvinylpyrrolidone (Kollidon 25) and lactose (Tablettose 70) in the above given amounts are mixed and compressed into tablets. Optionally, the asenapine maleate can be micronized prior to producing the tablets (see example 2d)).

Example 9:

Hygroscopicity of several dosage forms was determined as described in European Pham. Section 5.11. In particular, the samples (e.g. tablets) were weight in a weighing vessel and stored in a desiccator (containing a saturated ammonium chloride solution) for 24h at room temperature. The percentage increase in mass was calculated according the described formula:

$$m\ [\%] = [(m_3 - m_2) / (m_2 - m_1)] \times 100$$

m    increase in mass [%]
$m_1$    mass weighing vessel [mg]
$m_2$    mass weighing vessel + mass sample [mg]
$m_3$    mass weighing vessel + mass sample after 24h [mg]

The dosage form based on example 16 of WO2006/106135 is commercially available under the trade name Sycrest®. In the following, said conventional dosage form was compared to the dosage forms according to present examples 4 and 5. For each dosage form, five individual batches were evaluated. The following table summarizes the results:

| | Increase in mass [%] | | | | | |
|---|---|---|---|---|---|---|
| | Batch 1 | Batch 2 | Batch 3 | Batch 4 | Batch 5 | **Mean value** |
| **Sycrest®** | 4.24 | 4.59 | 4.32 | 4.59 | 3.94 | **4.32** |
| **Example 4** | 0.85 | 0.82 | 0.69 | 0.79 | 0.77 | **0.78** |
| **Example 5** | 0.69 | 0.64 | 1.18 | 0.85 | 0.93 | **0.86** |

The dosage forms according to examples 4 and 5 were significantly less hygroscopic than the conventional freeze-dried dosage form available under the trade name Sycrest® comprising orthorhombic asenapine maleate. In particular, the inventive dosage forms took up less than 0.9 w% water from about 80 % relative humidity at about 25 °C, whereas the dosage form available under the trade name Sycrest® took up about 4.3 w% water from about 80 % relative humidity at about 25 °C (as measured after 24 hours according to item 5.11. of the European Pharmacopoeia).

Example 10:

As noted above, a dosage form that is based on example 16 of WO2006/106135 is commercially available under the trade name Sycrest®, and comprises orthorhombic and amorphous asenapine maleate. Polymorph stability (i.e. crystallinity grade) ofthis dosage form was determined according to item 2.9.33 of the Ph.Eur. In particular, the samples were measured prior to and after determination of hygroscopicity. X-Ray diffraction patterns were obtained using a X'Pert PRO MPD system (PANalytical B.V., Netherlands) operating at 45 kV and 40 mA. Diffractogramms were recorded using following measurement parameters:

| XRPD - Parameter | |
|---|---|
| Scan range [°2Th] | 5.0 - 45.0 |
| Step size [°2Th] | 0.0084 |
| Time per step [sec] | 60 |
| Incident beam path | Soller slit: 0.02 rad, Mask: 15 mm, PDS: automatic, irradiated length 20.0 mm |
| Diffracted beam path | Soller slit: 0.02 rad PDS: automatic, irradiated length 20.0 mm |

The samples were measured on a rotating sample disk of Si with a speed of 1 rpm. Data evaluation was done with the help of X'Pert HighScore Plus® software according the following formula:

**% crystallinity = 100 A / ( A + B - C)**
wherein
A =          total area of the peaks arising from diffraction from the crystalline fraction of the sample;
B =          total area below area A;
C =          background area (due to air scattering, fluorescence, equipment, etc.).

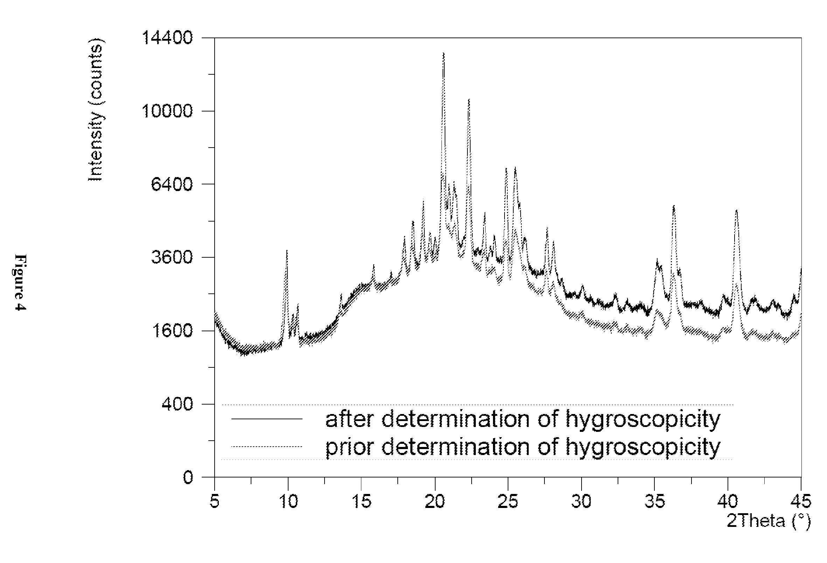

The results are qualitatively depicted in Figure 4 (bottom diffractogram: 5 mg of the dosage form commercially available under the trade name Sycrest® prior to determination of hygroscopicity (80% rH, 24h); top diffractogram: 5 mg of the dosage form commercially available under the trade name Sycrest® after determination of hygroscopicity (80% rH, 24h)). After storing the sample at 80% rH for 24h crystallinity significantly increased (higher area under the peaks), i.e. amorphous asenapine maleate at least in part recrystallized into the orthorhombic form.

In particular, it has been found that after exposing the dosage form based on example 16 of WO2006/106135 (commercially available under the trade name Sycrest®) to about 80 % relative humidity and about 25 °C for 24 hours, the dosage form comprising orthorhombic and amorphous asenapine maleate showed a significant increase of its crystallinity grade. A quantitative analysis of the diffractograms revealed that the crystallinity grade approximately increased about 18 % (mean value over five batches).

Example 11:

As noted above, a dosage form that is based on example 16 of WO2006/106135 is commercially available under the trade name Sycrest®, and comprises orthorhombic and amorphous asenapine maleate. Chemical stability (i.e. degradation products) of this dosage form was compared to that of the dosage form according to present example 4. The following table summarizes the experimental setup:

| chromatography parameters | HPLC- device: |  |  |
|---|---|---|---|
|  | pump | → | HP 1200 |
|  | autosampler | → | HP 1200 |
|  | detector | → | HP 1200 |
|  | or device with comparable construction |  |  |
| pre-column | ChromGuard C18 |  |  |

| column | XBridge C18 2,5 μm |
|---|---|
| column dimensions | 50 x 3,0 mm |
| column temperature | 25°C |
| temperature autosampler | room temperature |
| solvent A | 20 mM potassium dihydrogen phosphate-buffer pH 3,0<br>⇒*) gradient |
| solvent B | acetonitrile<br>⇒*) gradient |
| *) gradient | time (min)    % solvent A    % solvent B<br>0      90      10<br>20      10      90<br>21      90      10<br>26      90      10 |
| flow | 1,0 ml / min |
| injection volume | 10 μl |
| wavelength | 270 nm for assay<br>230 nm for test on impurities |
| standard solutions | 0,5 mg/ml asenapine for assay<br>0,001 mg/ml asenapine for test on impurities |
| sample solution | 0,5 mg/ml asenapine for assay/ for test on impurities |
| solvent composition for standard and sample solutions | methanol/water HPLC-quality 9:1 |
| sample preparation | samples are dissolved in water, treated in an ultrasonic bath, shaken, filled up with methanol and filtered. |

After storage of the dosage forms (in Alu blisters) for 4 weeks (60 °C, 75 % rh), the inventive dosage form showed an increase of degradation products (i.e. impurities [%]) of only about 0.45 %, whereas the dosage form commercially available under the trade name Sycrest® showed an increase of degradation products (i.e. impurities [%]) of about 1.5 %. Hence, it has been found that the dosage form of the present invention is chemically more stable than the conventional freeze-dried dosage forms comprising orthorhombic asenapine maleate. In particular, it has been unexpectedly found that the inventive dosage form comprising microcrystalline monoclinic asenapine maleate showed a significantly lower increase of degradation products than the conventional freeze-dried dosage forms after long-term storage.

**Claims**

1. A compressed pharmaceutical dosage form intended for sublingual or buccal administration and capable of being rapidly disintegrated, the compressed pharmaceutical dosage form containing monoclinic asenapine maleate in a crystalline form and at least one further excipient, wherein the particle size distribution of monoclinic asenapine maleate is **characterized by** a d95 of less than 75 μm, and wherein the crystalline form contains less than 5 % w/w of asenapine maleate in the crystalline orthorhombic form, and optionally contains no detectable amount of asenapine maleate in the crystalline orthorhombic form.

2. The compressed pharmaceutical dosage form according to claim 1, wherein the asenapine maleate is **characterized by** an X-ray powder diffraction pattern not comprising a peak at the 2- theta angle of 15.7 ± 0.2°, optionally **characterized by** an X-ray powder diffraction pattern substantially in accordance with figure 1.

3. The compressed pharmaceutical dosage form according to claim 1 or 2, wherein the asenapine maleate in the monoclinic form has a particle size distribution **characterized by** a d95 of at most 50 μm, optionally at most 30 11m.

4. The compressed pharmaceutical dosage form according to any of the preceding claims, wherein the asenapine maleate in the monoclinic form is present in an amount of from 5 to 40 % w/w, optionally from 10 to 30 % w/w.

5. The compressed pharmaceutical dosage form according to any of the preceding claims, wherein the at least one further excipient is a disintegrant, optionally selected from the group consisting of cross-linked carboxymethylcellulose sodium, cross-linked polyvinylpyrrolidone, pregelatinized starch, calcium carboxymethyl cellulose, sodium alginate, sodium starch glycolate, crospovidone, croscarmellose sodium and low substituted hydroxyl propyl cellulose.

6. The compressed pharmaceutical dosage form according to any of the preceding claims, wherein the at least one further excipient is a filler, optionally selected from the group consisting of lactose, mannitol, sorbitol, xylitol, powdered cellulose, microcrystalline cellulose, calcium carbonate, dibasic calcium phosphate and any mixture thereof.

7. The compressed pharmaceutical dosage form according to any of the preceding claims, wherein the at least one further excipient is a lubricant, optionally selected from the group consisting of calcium stearate, magnesium stearate, sodium stearyl fumarate, stearic acid and stearic acid salts.

8. The compressed pharmaceutical dosage form according to any of the preceeding claims having a crushing strength of from 5 N to 50 N, preferably of from 7 N to 40 N, more preferably from 7 N to 30 N, most preferably from 7 N to 20 N.

9. The compressed pharmaceutical dosage form according to any of the preceeding claims, wherein the disintegration time measured according to item 2.9.1, Test A of the European Pharmacopoeia is less than 1 min, optionally less than 30 s.

10. The compressed pharmaceutical dosage form according to any of the preceeding claims, wherein the compressed pharmaceutical dosage form is intended for sale in a country having areas with an Af or Am climate according to the K6ppen-Geiger climate classification.

11. A method for the preparation of a compressed pharmaceutical dosage form, optionally the compressed pharmaceutical dosage form according to any one of claims 1 to 10, the method comprising:

a) mixing a crystalline form of monoclinic asenapine maleate with at least one further excipient, wherein the particle size distribution of monoclinic asenapine maleate is **characterized by** a d95 of less than 75 μm, and wherein the crystalline form contains less than 5 % w/w of asenapine maleate in the crystalline orthorhombic form, and optionally contains no detectable amount of asenapine maleate in the crystalline orthorhombic form; and
b) compressing the mixture obtained from a) into a compressed pharmaceutical dosage form.

12. A compressed pharmaceutical dosage form obtainable by the method of claim 11.

13. A container comprising a compressed pharmaceutical dosage form containing microcrystalline monoclinic asenapine maleate, optionally comprising the compressed pharmaceutical dosage form according to any of claims 1 to 10 or 12, wherein the container is prepared from a material having a permeability for water vapor as measured according to DIN 53122 of from 1,0 g * m$^{-2}$ * d$^{-1}$ to 5000 g * m$^{-2}$ * d$^{-1}$.

14. The container of claim 13, which is a blister package, wherein the blister is made from polypropylene, polyvinylidenchloride and/or polyvinylchloride.

15. The container according to any of the preceding claims, wherein the container is intended for sale in a country having areas with an Af or Am climate according to the Köppen-Gelger climate classification.

Figure 1

**Figure 2**

**Figure 3**

**Figure 4**

Europäisches
Patentamt

European
Patent Office

Office européen
des brevets

# EUROPEAN SEARCH REPORT

Application Number

EP 11 18 2256

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2011/159903 A2 (REDDYS LAB LTD DR [IN]; REDDYS LAB INC DR [US]; KATKAM SRINIVAS [IN];) 22 December 2011 (2011-12-22) * claims 1,2 * * page 13, line 4 * * page 42, line 12 * * page 43, lines 12,27 * * page 26, line 27 * ----- | 1-15 | INV. A61K9/00 A61K9/20 A61K31/407 |
| A | FUNKE C W ET AL: "PHYSICO-CHEMICAL PROPERTIES AND STABILITY OF TRANS-5-CHLORO-2- METHYL-2,3,3A, 12B-TETRAHYDRO-1H-DIBENZ-2,3:6,70 OXEPINO-4,5-C) PYRROLIDINE MALEATE", ARZNEIMITTEL FORSCHUNG. DRUG RESEARCH, ECV EDITIO CANTOR VERLAG, AULENDORF, DE, vol. 40, no. 5, 1 May 1990 (1990-05-01), pages 536-539, XP000561359, ISSN: 0004-4172 * the whole document * ----- | 1-15 | |
| A | WO 2006/106135 A1 (ORGANON IRELAND LTD [IE]; HEERES GERHARDUS JOHANNES [NL]) 12 October 2006 (2006-10-12) * claims 1-13 * ----- | 1-15 | TECHNICAL FIELDS SEARCHED (IPC) A61K |

The present search report has been drawn up for all claims

| Place of search | Date of completion of the search | Examiner |
|---|---|---|
| Munich | 17 February 2012 | Giacobbe, Simone |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 11 18 2256

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2012

| Patent document cited in search report | Publication date | Patent family member(s) | Publication date |
|---|---|---|---|
| WO 2011159903 A2 | 22-12-2011 | NONE | |
| WO 2006106135 A1 | 12-10-2006 | AR      056306 A1 | 03-10-2007 |
| | | AT      370954 T | 15-09-2007 |
| | | AT      418556 T | 15-01-2009 |
| | | AU  2006231617 A1 | 12-10-2006 |
| | | BR   PI0609741 A2 | 27-04-2010 |
| | | CA     2603509 A1 | 12-10-2006 |
| | | DE 602006000080 T2 | 15-05-2008 |
| | | DK     1710245 T3 | 03-12-2007 |
| | | DK     1917267 T3 | 14-04-2009 |
| | | DO   P20060082 A | 30-11-2006 |
| | | EP     1710245 A1 | 11-10-2006 |
| | | EP     1917267 A1 | 07-05-2008 |
| | | ES     2293626 T3 | 16-03-2008 |
| | | ES     2318742 T3 | 01-05-2009 |
| | | HN  2006014095 A | 19-08-2010 |
| | | HR    20070429 T3 | 30-11-2007 |
| | | JP  2008534656 A | 28-08-2008 |
| | | KR  20080005253 A | 10-01-2008 |
| | | NZ      562124 A | 30-07-2010 |
| | | PE    13112006 A1 | 09-02-2007 |
| | | PT     1710245 E | 03-10-2007 |
| | | PT     1917267 E | 04-02-2009 |
| | | SI     1710245 T1 | 31-12-2007 |
| | | SI     1917267 T1 | 30-04-2009 |
| | | SV   2006002473 A | 24-11-2009 |
| | | UY      29459 A1 | 30-11-2006 |
| | | WO  2006106135 A1 | 12-10-2006 |

EPO FORM P0459

For more details about this annex : see Official Journal of the European Patent Office, No. 12/82

## REFERENCES CITED IN THE DESCRIPTION

### Patent documents cited in the description

- US 4145434 A
- WO 2006106135 A
- WO 9523600 A
- EP 1710245 A1
- EP 1710245 A
- WO 20061061 A
- WO 2008003460 A, Ullman

### Non-patent literature cited in the description

- **FUNKE et al.** *Arzneim.-Forsch./Drug Res.,* 1999, vol. 40 (5), 536-539
- **HILFIKER et al.** Polymoprhism in the Pharmaceutical Industry. WILEY-VHC Verlag GmbH & Co. KGaA, 2006